# EUROPEAN PATENT APPLICATION

(11) **EP 4 338 760 A1**
(43) Date of publication of application: **20.03.2024**
(21) Application number: 22195971.1
(22) Date of filing: 15.09.2022
(51) Int. Cl.: A61L 2/10, B64D 11/02, B64D 11/04, B64C 1/18

(54) **UV FLOOR COVERING**

(71) Applicant: B/E Aerospace, Inc., Winston-Salem, NC 27105 (US)
(72) Inventor: TUICO, Roderico, Tanauan City, 4232 (PH); DELA CRUZ, Ma Anicia Bautista, Tanauan City, 4232 (PH); ESPIRITU, Jose Federico Soriano, Tanauan City, 4232 (PH)
(74) Representative: Dehns

(57) **Abstract**

A floor covering (1) for an aircraft interior including a substrate (9) and a plurality of UV lights (8) distributed across the surface of the substrate (9). The floor covering (1) further includes at least one sensor (6) configured to detect the presence of an object (15) on the substrate (9). At least one of the plurality of UV lights (8) emits UV radiation when the at least one sensor (6) detects the presence of an object (15) on the substrate (9). The UV lights (8) may be configured to act as a germicide, and the object (15) may be a foot or footwear. Therefore, the floor covering (1) may be provided as a safety measure for an aircraft.

## Description

### Technical Field

This disclosure relates to a floor covering for an aircraft interior. In particular, the disclosure relates to a floor covering with UV lights.

### Background Art

Aircraft are busy spaces with a large number and high turnover of passengers. In order to provide a safe environment for passengers and staff, it is desirable for aircraft to be thoroughly cleaned and sanitised. This has recently been highlighted by the Covid-19 pandemic; however, there are many other viruses, bacteria and pathogens that may be present on aircraft and which may be harmful to human health. The number of any such pathogens should be kept to a safe level inside aircraft.

Typically, aircraft interiors are cleaned using chemicals such as chlorine and alcohol. However, in the long-term, people who come into close contact with the chemicals may experience harmful effects to their health. The chemicals are also a consumable which must be purchased regularly, increasing operating costs of the aircraft. The present disclosure aims to address some of these problems by providing an improved floor covering.

### Summary of the Disclosure

According to a first aspect of the present disclosure, there is provided a floor covering for an aircraft interior, comprising:
a substrate;
a plurality of UV lights distributed across the surface of the substrate; and
at least one sensor configured to detect the presence of an object on the substrate;
wherein at least one of the plurality of UV lights emits UV radiation when the at least one sensor detects the presence of an object on the substrate.

The present disclosure provides a floor covering having a substrate and UV lights. The floor covering further includes a sensor that detects (e.g. the weight of) an object on the substrate. When the object is detected on the substrate, the UV lights emit UV radiation.

In some examples of the present disclosure, the UV radiation may be of a suitable wavelength and intensity to kill or deactivate pathogens. By providing such UV lights in a floor covering, some examples of the present disclosure may enable objects on the floor covering to be sanitised effectively by the UV radiation emitted from the UV lights.

The substrate may be any material suitable for use as a floor covering in an aircraft. Therefore, the substrate may be durable and/or lightweight. For example, the substrate may comprise a laminate, vinyl, rubber, wool or acrylic.

In some examples, the substrate comprises a durable outer layer. The durable outer layer may comprise any suitable and desired material. In some examples, at least some of the UV lights are located below the durable outer layer. In some examples, the durable outer layer is at least partially transparent to UV radiation. This allows at least some of the UV radiation emitted by the UV lights to escape through the durable outer layer and into the environment surrounding the floor covering.

In some examples, the plurality of UV lights are homogeneously distributed across the surface of the floor covering. in some examples the UV lights are distributed (e.g. homogeneously) across substantially the entire surface of the floor covering. The UV lights may be distributed in any suitable and desired manner across the surface of the floor covering. For example, they may be scattered in a random manner or they may form a repeating pattern, e.g. a tiled or grid formation. Distributing the UV lights in this manner helps to increase the likelihood that an object placed anywhere on the floor covering will be irradiated when the UV lights emit radiation.

In some examples, the substrate comprises a plurality of holes, and the plurality of UV lights are situated in the plurality of holes. The holes in the substrate may have substantially the same dimensions as the UV lights. The UV lights may be held in place by an interference fit with the sides of the holes, or by additional means such as an adhesive. Placing the UV lights into holes in the substrate may be advantageous in some examples of the present disclosure because it helps to enable the substrate and the UV lights to be manufactured separately and combined into one product at a later stage.

The UV lights may have any depth (e.g. vertical position) in the holes, but preferably their depth is such that the top surface of the UV light is substantially level with or (e.g. partially) above the top surface of the substrate. This position may help to increase the amount of UV radiation that is able to escape into the environment above the substrate.

In some examples, the plurality of UV lights are embedded into the substrate. The UV lights being embedded means that they are at least partially surrounded by the material of the substrate. The UV lights may be embedded between layers of the floor covering, for example, between the substrate and durable outer layer, when present.

In some examples, the UV lights may be embedded into a layer of the substrate during manufacture. In some examples, this may be achieved by the substrate being moulded around the UV lights.

In some examples, the plurality of UV lights form a mesh with the substrate. In some examples, the substrate may be a carpet formed from interwoven fibres, e.g. of wool or acrylic. In some examples, the UV lights are connected to a power source by at least one wire, which may be arranged to form a mesh with the material of the substrate, e.g. the wire may be treated as a fibre. The method of forming the mesh may be any suitable and desired method, for example, weaving, knotting, twisting or knitting.

The UV lights may be arranged to emit any suitable and desired wavelength of UV light. In some examples, the UV lights are configured to emit UVC radiation. UVC radiation has a wavelength between 100 nm and 280 nm. The emission spectrum may take any suitable form, e.g. a narrow band or a broad band of wavelengths may be used.

In some examples, the UV lights are configured to act as a germicide. UV radiation may help to kill or deactivate pathogens such as bacteria, viruses and mould by disrupting their DNA. The result is to reduce the number, or effectiveness, of the pathogens. UV radiation may be effective as a germicide for pathogens located in air, water, or on surfaces. The effectiveness of UV radiation as a germicide may be related to the intensity of the radiation and the duration of exposure of the pathogens to the radiation. UV radiation is more effective as a germicide the longer the exposure and the more intense the radiation. Therefore, the intensity of the UV radiation and the length of time that the UV lights emit UV radiation may be chosen such that the desired effect is achieved.

In some examples, the plurality of UV lights comprise light emitting diodes. Any suitable and desired light emitting diode (LED) may be used. LED lights may be advantageous in some examples of the present disclosure owing to their small size, low power consumption and safety. LED lights may optionally have very narrow waveband of radiation. Therefore, in some examples, the wavelength or emission spectrum may be chosen for its germicidal properties.

In some examples, the plurality of UV lights are coupled to a plurality of fibre optic cables. Any suitable and desired source of UV light may be coupled to the fibre optic cable. In some examples, the fibre optic cables may be used to transmit light across the floor covering. In some examples, the fibre optic cables may be used to split the radiation emitted from a single UV light into several smaller outputs distributed across the substrate.

In some examples, the plurality of UV lights comprise UV lamps. Any suitable and desired UV lamp may be used. For example, the UV lights may comprise a low-pressure mercury lamp, a pulsed xenon lamp, a cold cathode lamp or a coil filament lamp.

In some examples, the sensor is configured to detect the force of an object on the substrate. The sensor may be any suitable or desired type of sensor. The sensor may be configured to detect any suitable physical measurement that relates to the force that an object exerts on the substrate. For example, the sensor may comprise a pressure sensor, a stress sensor, a strain sensor, a load sensor or a weight sensor.

Any number of sensors may be used. For example, a single sensor may cover the entire area of the substrate or several smaller sensors may be used. When the floor covering comprises a plurality of sensors, the sensors may be arranged in any suitable manner. For example, the sensors may be placed in a regular tiled arrangement across the substrate or they may be placed only in the areas of highest footfall. The plurality of sensors may be used independently or some may be used together to achieve a level of redundancy, e.g. to compare measurements between sensors to verify that the measurement is correct.

The sensor(s) may be placed below the substrate or may form part of the substrate, for example the sensor(s) may be embedded in the substrate or between layers of the substrate.

The output of the sensor(s) may be any suitable form. For example, the output may be binary or continuous. A binary output switches between a high and a low state, with high for example indicating the presence of an object and low indicating that no object is present. A continuous output may take any number of values, and may be proportional to the amount of force being exerted on the substrate by the object.

In some examples, the object is a foot or footwear. In some examples, the floor covering may be situated in areas where aircraft passengers are likely to step. For example, the entrance to the aircraft, the galleys, the aisles or the bathrooms.

In some examples, the UV lights are configured to irradiate the foot or footwear, and optionally act as a germicide to the pathogens on the surface of the foot or footwear. The object may be any number of feet or footwear, as it will be understood that any number of people may stand on the floor covering at one time.

In some examples, the floor covering is configured to energise at least one (e.g. all) of the plurality of UV lights when the at least one sensor detects the presence of an object on the substrate.

According to a second aspect of the present disclosure, there is provided a system comprising a floor covering and a power source, wherein the power source is connected to the plurality of UV lights and is arranged to energise the plurality of UV lights, e.g. when the at least one sensor detects the presence of an object on the substrate. The floor covering may include any of the features discussed herein.

In some examples, the system comprises a switch. The power source may be connected to the plurality of UV lights via the switch. The at least one sensor may be connected to the switch, e.g. the at least one sensor may be arranged to actuate the switch, e.g. when the at least one sensor detects the presence of an object on the substrate.

The power source may be any suitable or desired power source. For example, the power source may comprise a solar cell, a battery, a mains connection, a generator or a piezoelectric material. The power source may also be arranged to power other components of the aircraft. The power source may be arranged to provide power in any suitable form, for example AC or DC current. The power source may comprise any combination of converters and transformers in order to be compatible with the floor covering.

In some examples, the power source is a solar cell. The solar cell may be any suitable and desired solar cell. In some examples, the solar cell may comprise part of a window covering of the aircraft, for example integrated into a blind. In some examples, the solar cell may form part of an external panel of the aircraft.

In some examples, the power source is a battery of the aircraft. The battery may be any suitable and desired battery. In some examples, the battery may be configured to supply other components of the aircraft. In some examples, the battery may further comprise a voltage converter, which converts the output voltage of the battery to a supply voltage for the floor covering.

In some examples, the UV lights are configured to cease emitting UV radiation when the at least one sensor detects that the object is no longer present on the substrate. In some examples, the sensor continues to monitor the force that the object is exerting on the substrate after the object is first detected. When a reduction in measured force indicates that the object is no longer present on the substrate, the sensor may send a further signal to the UV lights which causes them to cease emitting UV radiation. Some examples of this disclosure may prevent the UV lights from emitting an excessive amount of UV radiation, thereby reducing energy consumption and extending the lifetime of the bulb.

In some examples, the UV lights are configured to stop emitting UV radiation after a particular period of time has elapsed. The particular period of time may be measured by a timer which begins counting when the UV lights start emitting radiation. In some examples, the particular period of time is no more than 20 seconds, optionally no more than 30 seconds, optionally no more than 1 minute, optionally no more than 2 minutes, optionally no more than 3 minutes, optionally no more than 5 minutes.

The particular period of time may be chosen to deliver a certain dose of radiation to the object that is sufficient to kill or deactivate a certain percentage of pathogens. The particular period of time may therefore be dependent on the intensity of the UV radiation, e.g. a shorter particular period may be chosen for more intense UV radiation. Some examples of this disclosure may prevent the UV lights emitting an excessive amount of UV radiation (e.g. more than the amount required to kill or deactivate the pathogens), in particular when the object remains on the substrate for an extended period of time, thereby helping to reduce energy consumption and extend the lifetime of the UV lights.

### Brief Description of the Drawings

Certain examples of the present disclosure will now be described with reference to the accompanying drawings in which:
Figure 1 is a schematic view of a floor covering and a solar cell power source in accordance with an example of the present disclosure;
Figure 2 is a schematic view of a floor covering and an aircraft battery power source in accordance with an example of the present disclosure;
Figures 3 a-d are schematic views of arrangements of the substrate of the floor covering and the UV lights in accordance with an example of the present disclosure; and
Figure 4 is a flow chart showing a method in accordance with an example of the present disclosure.

### Detailed Description

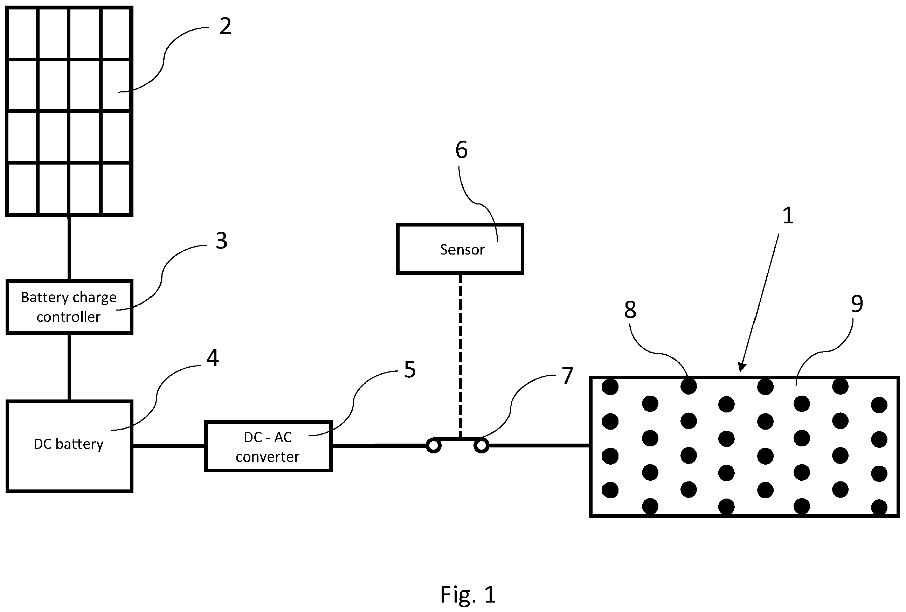

Figure 1 is a schematic view of a floor covering 1 and a solar cell 2 power source according to an example of the present disclosure.

The solar cell 2 is connected to a battery charge controller 3. The battery charge controller 3 is further connected to a DC battery 4. The DC battery 4 is further connected to a DC-AC converter 5.

The floor covering 1 of Figure 1 comprises a sensor 6 which is connected via a switch 7 to a plurality of UV lights 8. The UV lights 8 are distributed across the surface of the substrate 9 of the floor covering 1.

The solar cell 2 may be any suitable and desired solar cell. In some examples, the solar cell 2 may be part of a window covering of the aircraft. The battery charge controller 3 regulates the charge produced by the solar cell 2. For example, it may prevent surges in charge and current.

The DC battery 4 is arranged to store at least some of the charge produced by the solar cell 2. This helps to accommodate inconsistencies from the charge produced by a solar cell, that may even be present with the battery charge controller 3 in place. The DC-AC converter 5 coverts the direct current into an alternating current, which is suitable for energising the UV lights 8.

In this example, the UV lights 8 are homogeneously distributed across the surface of the substrate 9 of the floor covering 1. However, it will be appreciated that in other examples the UV lights 8 may be distributed in another manner across the surface of the substrate 9.

The sensor 6 is configured to detect the force of an object on the substrate 9 of the flooring. In this example, the sensor 6 is directly connected to the switch 7. However, it will be appreciated that other suitable connections may be used between the sensor 6 and the switch 7. For example, the sensor 6 and the switch 7 may be further connected to a processor which processes the signal from the sensor 6 and sends a further signal to the switch 7.

The switch 7 is in the normally-open position, meaning that the power source is disconnected from the UV lights 8. Upon detecting force, the sensor 6 is configured to actuate the switch 7 to the closed position, thereby connecting the solar cell 2 and the UV lights 8. The solar cell 2 is configured to energise the UV lights 8 to emit UV radiation.

Therefore, the UV lights 8 are configured to emit UV radiation only when an object is detected on the substrate 9 of the flooring. This has advantages over a system in which the UV lights emit UV radiation at all times.

First, exposure to potentially harmful UV radiation may be reduced for people nearby the floor covering. This may be particularly advantageous for applications in, e.g., the interior floor of an aircraft, where people may spend an extended period, e.g. in the cabin of an aircraft.

Second, the amount of energy required to power the UV lights maybe reduced, and the lifetime of the UV lights may be prolonged as a result of their reduced operating period.

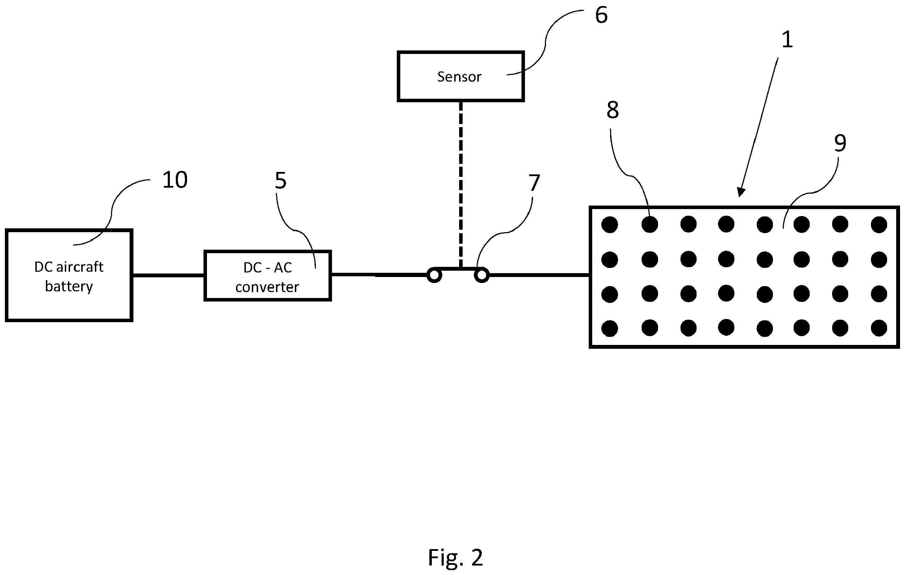

Figure 2 is a schematic view of a floor covering 1 and an aircraft battery 10 power source according to an example of the present disclosure. The example in Figure 2 is configured to operate in the same manner as the example shown in Figure 1. However, in this example the power source is a DC aircraft battery 10.

In some examples, the system may further comprise a DC-DC converter connected between the aircraft battery 10 and the DC-AC converter 5. In such examples, the DC-DC converter may be arranged to reduce the voltage from a high-voltage aircraft battery 10, such that the voltage output is suitable for use in end applications such as the floor covering 1 of this example.

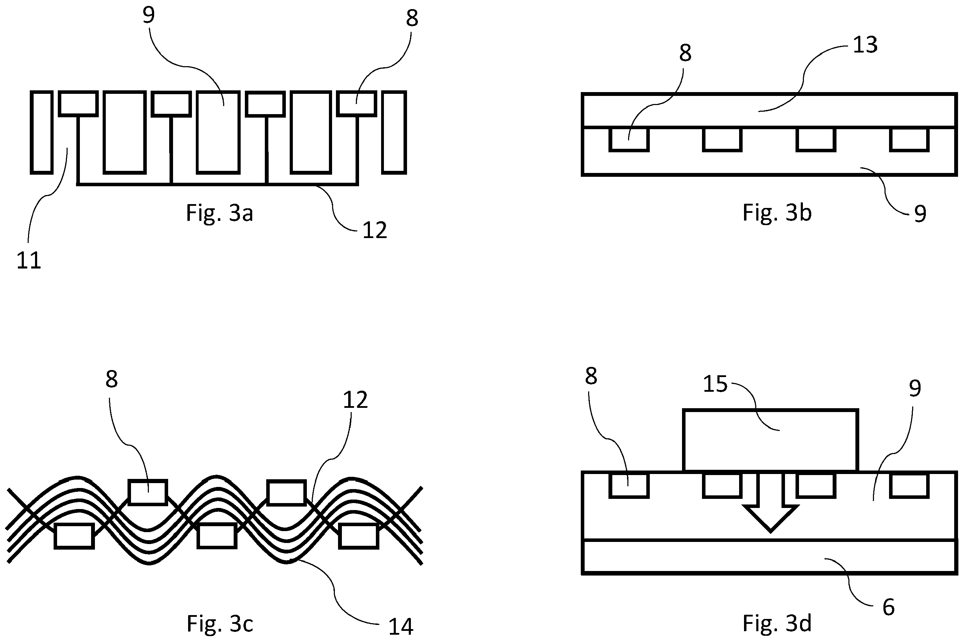

Figures 3 a-d are schematic views of arrangements of UV lights 8 and a substrate 9 according to an example of the present disclosure.

Figure 3a shows an example whereby the substrate 9 of the floor covering has a number of holes 11 and the UV lights 8 are situated in the holes 11. In this example, the UV lights 8 are connected to the power source by wires 12. The UV lights 8 may be connected in any suitable and desired way, for example in series or in parallel.

The holes 11 in the substrate have substantially the same dimensions as the UV lights 8. The UV lights 8 may be held in place by an interference fit with the sides of the holes 11, or by additional means such as an adhesive.

The UV lights 8 may have any vertical position in the holes 11, but preferably their vertical position is such that the top surface of the UV light 8 is substantially level with the top surface of the substrate 9. In this position, the maximum amount of UV radiation is able to escape into the environment above the substrate 9.

Figure 3b shows an example in which the UV lights 8 are embedded into the substrate 9. In this example, the substrate 9 additionally comprises a durable outer layer 13. The durable outer layer 13 may be at least partially transparent to UV wavelengths of light, such that the UV radiation emitted from the UV lights 8 is able to penetrate this layer. However, it will be appreciated that the durable outer layer 13 may not always be present.

The UV lights 8 may be embedded in the substrate 9 during manufacture of the floor covering 1. In some examples, the UV lights 8 may be placed at the bottom of a mould and the material for the substrate 9 is poured into the mould over the UV lights 8. However, it will be appreciated that there are other suitable methods to embed the UV lights 8 into the substrate 9.

The UV lights 8 may be embedded at any vertical position in the substrate 9, but preferably their vertical position is such that the top surface of the UV light 8 is substantially level with the top surface of the substrate 9. In this position, the maximum amount of UV radiation is able to escape into the environment above the substrate 9.

Figure 3c shows an example in which the substrate 9 of the floor covering 1 is a fibrous material 14. In such examples, the substrate 9 may be a carpet, and may be formed from interwoven fibres of wool or acrylic.

In some examples, the UV lights 8 are connected to the power source by at least one wire 12, which may be treated as a fibre and arranged to form a mesh with the material of the substrate 9. The method of forming the mesh may be any method known in the art, for example, weaving, knotting, twisting or knitting.

Figure 3d shows an example in which the sensor 6 is connected to the substrate 9. In some examples, the sensor 6 is embedded into the substrate 9. In such examples, the sensor 6 may be embedded in the substrate 9 during manufacture of the floor covering 1. In this example, the sensor 6 is shown at the bottom of the substrate 9; however, it will be appreciated that the sensor 6 may be embedded at any vertical height with respect to the substrate 9.

In some examples, the sensor 6 may be a separate component to the substrate 9. In such examples, the sensor 6 may be placed between the floor and the substrate 9, for example when the floor covering 1 is being installed. In such examples, an additional material such as an adhesive may be used to secure the sensor 6 to the substrate 9 and/or to the floor.

In this example, the sensor 6 extends across the width of the substrate 9. However, it will be appreciated that the sensor 6 may not extend over the entire area of the substrate 9. For example, the sensor 6 may be formed of several smaller sensors tiled together and spaced at intervals across the area of the substrate 9. In some examples, the sensor 6 may be placed in areas of particularly high footfall or at the boundary between rooms or areas, for example at the entrance to the aircraft or the door of a bathroom.

In Figure 3d, an object 15 is shown on top of the substrate 9. The arrow represents the force that the object 15 exerts on the substrate 9. The sensor 6 is configured to detect the force exerted by the object 15 on the substrate 9. However, it will be appreciated that the sensor 6 could also be configured to carry out other suitable measurements such as the force, weight, or strain that the object 15 exerts upon the substrate 9.

In some examples, the sensor 6 may compare the measured force to a threshold, so that the UV lights 8 are not energised by very small objects such as dust and debris. In some examples, the sensitivity of the sensor 6 may be chosen such that it does not cause the switch 7 to close for very small objects.

In some examples, the object 15 being detected is a person. In some examples, the object 15 is a person's foot or footwear. In such examples, the UV lights 8 may be configured to act a germicide, such that the UV radiation disinfects the person's foot or footwear.

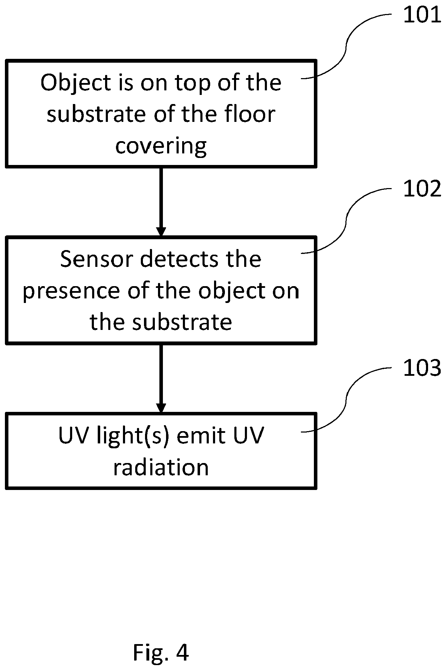

Figure 4 is a flow chart showing a method according to an example of the present disclosure, which will now be described with reference to Figures 1-3d. In step 101, an object 15 is on top of substrate 9 of the flooring. In step 102, the sensor 6 detects the presence of the object 15 on the substrate 9. In step 103, the UV light(s) 8 emit UV radiation. It will be appreciated that the method may include any of the features mentioned above.

When the object 15 is on top of the substrate 9, it exerts a downwards force into the substrate 9. The sensor 6 is configured to detect the force exerted onto the substrate 9 by the object 15. Upon detecting the force, the sensor 6 is configured to actuate a switch 7 to the closed position, thereby connecting the power source (e.g. solar cell 2 or aircraft battery 10) and the UV lights 8. The power source 2,10 is configured to energise the UV lights 8 to emit UV radiation. According to this method, the UV lights 8 only emit UV radiation when an object 15 has been detected on top of the substrate 9.

It will be seen from the above that, in at least some examples of the present disclosure, providing sanitising means in a floor covering that people may stand on helps to reduce the number of pathogens on people's feet or footwear. Many personal hygiene measures exist to reduce the number of pathogens on people's hands, for example, regularly washing hands and using hand gel. However, hygiene measures to clean people's feet or footwear are not commonly used. Examples in accordance with the present disclosure help to provides a solution to this problem.

By providing UV lights that are configured to emit UV radiation when the sensor detects an object on the floor covering, there is no need for a person to switch the lights on. Examples in accordance with the present disclosure thus help to provide a means for cleaning that requires no additional effort from a human.

By providing a means to reduce the number of pathogens on people's feet or footwear, examples in accordance with the present disclosure help to increase consumer confidence in the safety of air travel, which has become a more common problem since the Covid-19 pandemic.

While specific examples of the disclosure have been described in detail, it will be appreciated by those skilled in the art that the examples described in detail are not limiting on the scope of the disclosure. The scope of protection is defined by the appended claims.

## Claims

1. A floor covering for an aircraft interior, comprising:
a substrate;
a plurality of UV lights distributed across the surface of the substrate; and
at least one sensor configured to detect the presence of an object on the substrate;
wherein at least one of the plurality of UV lights emits UV radiation when the at least one sensor detects the presence of an object on the substrate.

2. The floor covering as claimed in claim 1, wherein the plurality of UV lights are homogeneously distributed across the surface of the floor covering.

3. The floor covering as claimed in claims 1 or 2, wherein the substrate comprises a plurality of holes, and the plurality of UV lights are situated in the plurality of holes.

4. The floor covering as claimed in claim 1 or 2, wherein the plurality of UV lights are embedded into the substrate.

5. The floor covering as claimed in claim 1 or 2, wherein the plurality of UV lights form a mesh with the substrate.

6. The floor covering as claimed in any preceding claim, wherein the UV lights are configured to act as a germicide.

7. The floor covering as claimed in any preceding claim, wherein the plurality of UV lights comprise light emitting diodes.

8. The floor covering as claimed in any preceding claim, wherein the plurality of UV lights are coupled to a plurality of fibre optic cables.

9. The floor covering as claimed in any preceding claim, wherein the sensor is configured to detect the force of an object on the substrate.

10. The floor covering as claimed in any preceding claim, wherein the object is a foot or footwear.

11. The floor covering as claimed in any preceding claim, wherein the UV lights are configured to cease emitting UV radiation when the at least one sensor detects that the object is no longer present on the substrate.

12. The floor covering as claimed in any of claims 1-10, wherein the UV lights are configured to cease emitting UV radiation after a particular period of time has elapsed.

13. A system comprising a floor covering as claimed in any preceding claim and a power source, wherein the power source is connected to the plurality of UV lights and is arranged to energise the plurality of UV lights.

14. The system as claimed in claim 13, wherein the power source is a solar cell.

15. The system as claimed in claim 13, wherein the power source is a battery of the aircraft.
